# EUROPEAN PATENT SPECIFICATION

(11) **EP 1 497 291 B1**
(45) Date of publication and mention of the grant of the patent: **22.11.2006**
(21) Application number: 03744860.2
(22) Date of filing: 25.03.2003
(51) Int. Cl.: C07D 471/04, A61K 31/395, A61P 25/00, C07D 215/46, C07D 215/40

(54) **QUINOLINE AND AZA-INDOLE DERIVATIVES AND THEIR USE AS 5-HT6 LIGANDS**
CHINOLIN- UND AZA-INDOLDERIVATE UND DEREN VERWENDUNG ALS 5-HT6 LIGANDEN
DERIVES DE QUINOLEINE ET D'AZA-INDOLE ET LEUR UTILISATION COMME LIGANDS 5-HT6

(30) Priority: 27.03.2002 GB 0207278; 27.03.2002 GB 0207281; 27.03.2002 GB 0207275; 27.03.2002 GB 0207282
(43) Date of publication of application: 19.01.2005
(73) Proprietor: GLAXO GROUP LIMITED, Greenford, Middlesex UB6 ONN (GB)
(72) Inventor: Johnson, Christopher Norbert, GlaxoSmithKline, Harlow, Essex CM19 5AW (GB); MacDonald, Gregor, James, Harlow, Essex CM19 5AW (GB); Mitchell, Darren Jason, GlaxoSmithKline, Harlow, Essex CM19 5AW (GB); Moss, Stephen Frederick, GlaxoSmithKline, Harlow, Essex CM19 5AW (GB); Thompson, Mervyn, GlaxoSmithKline, Harlow, Essex CM19 5AW (GB); Witty, David, GlaxoSmithKline, Harlow, Essex CM19 5AW (GB)
(74) Representative: Gibson, Mark
(86) International application number: PCT/EP2003/003195
(87) International publication number: WO 2003/080608

(56) References cited:
- WO-A-01/12629
- WO-A-99/65906

## Description

This invention relates to novel quinoline and aza indole compounds having pharmacological activity, processes for their preparation, to compositions containing them and to their use in the treatment of CNS and other disorders.

WO 98/27081 discloses a series of aryl sulphonamide compounds that are said to be 5-HT₆ receptor antagonists and which are claimed to be useful in the treatment of various CNS disorders. GB-2341549, WO 99/47516 and WO 99/65906 all disclose a series of indole derivatives that are claimed to have 5-HT₆ receptor affinity. JP 02262627 (Japan Synthetic Rubber Co) describes a series of substituted quinoline derivatives useful as wavelength converting elements. WO 01/83456 (Yamanouchi Pharmaceutical Co. Ltd) describes a series of bicyclic or tricyclic fused heteroaryl compounds with phosphatidylinositol 3-kinase activity. WO 00/42026 (Novo Nordisk) describes a series of quinoline and quinoxaline compounds for use as GLP-1 agonists. JP 08003144 (Chugai Pharmaceutical Co. Ltd.) describes a series of quinazoline and quinoline derivatives as potassium channel openers. WO 97/03069 (Glaxo Group Limited) and WO 96/09294 (The Wellcome Foundation Limited) both describe a series of substituted quinolines and quinazolines as protein tyrosine kinase inhibitors.

A structurally novel class of compounds has now been found which also possess affinity for the 5-HT₆ receptor. The present invention therefore provides, in a first aspect, a compound of formula (I) or a pharmaceutically acceptable salt thereof: 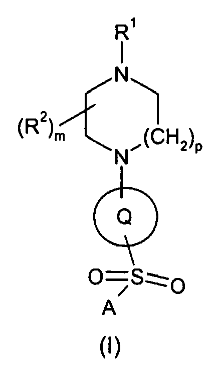 wherein
R¹ and R² independently represent hydrogen or C₁₋₆ alkyl or R¹ is linked to R² to form a group (CH₂)₂, (CH₂)₃ or (CH₂)₄;
p represents 1 or 2;
m represents an integer from 1 to 4, when m is an integer greater than 1, two R² groups may instead be linked to form a group CH₂, (CH₂)₂ or (CH₂)₃;
Q represents a group of formula (i), (ii), (iii) or (iv): 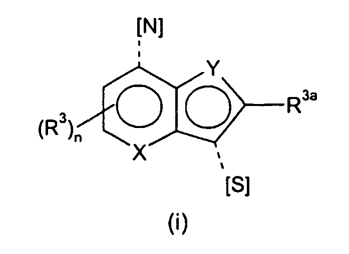 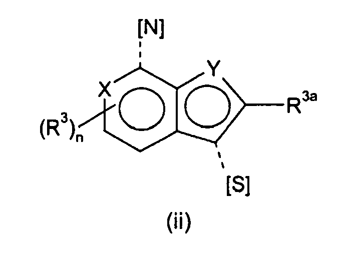 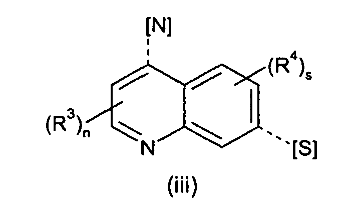 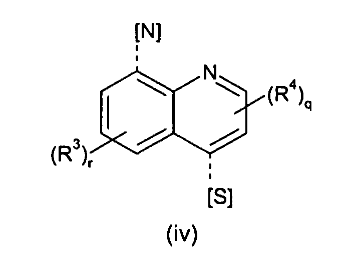 wherein [N] and [S] represent the attachment points for the groups 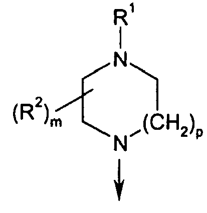 and 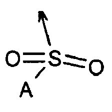 respectively;
one of X and Y represents -N= and the other represents -N(R⁵)-;
R³ and R⁴ independently represent hydrogen, halogen, cyano, -CF₃, -OCF₃, C₁₋₆ alkyl, C₁₋₆ alkoxy, C₁₋₆ alkanoyl or a group -CONR⁶R⁷;
R^{3a} and R⁵ independently represent hydrogen or C₁₋₆ alkyl;
R⁶ and R⁷ independently represent hydrogen or C₁₋₆ alkyl or together may be fused to form a 5- to 7- membered aromatic or non-aromatic heterocyclic ring optionally interrupted by an O or S atom;
n and q independently represent 1 or 2;
r and s independently represent an integer from 1 to 3;
A represents a group -Ar¹ or - Ar²Ar³;
Ar¹, Ar² and Ar³ independently represent an aryl group or a heteroaryl group, both of which may be optionally substituted by one or more (eg. 1, 2 or 3) substituents which may be the same or different, and which are selected from the group consisting of halogen, hydroxy, cyano, nitro, trifluoromethyl, trifluoromethoxy, C₁₋₆ alkyl, trifluoromethanesulfonyloxy, pentafluoroethyl, C₁₋₆ alkoxy, arylC₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, arylsulfonyl, arylsulfonyloxy, arylsulfonylC₁₋₆ alkyl, C₁₋₆ alkylsulfonamido, C₁₋₆ alkylamido, C₁₋₆ alkylsulfonamidoC₁₋₆ alkyl, C₁₋₆ alkylamidoC₁₋₆ alkyl, arylsulfonamido, arylcarboxamido, arylsulfonamidoC₁₋₆ alkyl, arylcarboxamidoC₁₋₆ alkyl, aroyl, aroylC₁₋₆ alkyl, arylC₁₋₆ alkanoyl, or a group CONR⁸R⁹ or SO₂NR⁸R⁹, wherein R⁸ and R⁹ independently represent hydrogen or C₁₋₆ alkyl or together may be fused to form a 5- to 7- membered aromatic or non-aromatic heterocyclic ring optionally interrupted by an O or S atom;
or solvates thereof.

Alkyl groups, whether alone or as part of another group, may be straight chain or branched and the groups alkoxy and alkanoyl shall be interpreted similarly. Alkyl moieties are more preferably C₁₋₄ alkyl, eg. methyl or ethyl. The term 'halogen' is used herein to describe, unless otherwise stated, a group selected from fluorine, chlorine, bromine or iodine.

The term "aryl" includes phenyl and naphthyl.

The term "heteroaryl" is intended to mean a 5-7 membered monocyclic aromatic or a fused 8-10 membered bicyclic aromatic ring containing 1 to 3 heteroatoms selected from oxygen, nitrogen and sulphur. Suitable examples of such monocyclic aromatic rings include thienyl, furyl, pyrrolyl, triazolyl, imidazolyl, oxazolyl, thiazolyl, oxadiazolyl, isothiazolyl, isoxazolyl, thiadiazolyl, pyrazolyl, pyrimidyl, pyridazinyl, pyrazinyl and pyridyl. Suitable examples of such fused aromatic rings include benzofused aromatic rings such as quinolinyl, isoquinolinyl, quinazolinyl, quinoxalinyl, cinnolinyl, naphthyridinyl, indolyl, indazolyl, pyrrolopyridinyl, benzofuranyl, benzothienyl, benzimidazolyl, benzoxazolyl, benzisoxazolyl, benzothiazolyl, benzisothiazolyl, benzoxadiazolyl, benzothiadiazolyl and the like. Heteroaryl groups, as described above, may be linked to the remainder of the molecule via a carbon atom or, when present, a suitable nitrogen atom except where otherwise indicated above.

It will be appreciated that wherein the above mentioned aryl or heteroaryl groups have more than one substituent, said substituents may be linked to form a ring, for example a carboxyl and amine group may be linked to form an amide group.

### For compounds of formula (I) wherein Q represents a group of formula (i):

Preferably, R¹ represents hydrogen or methyl, more preferably hydrogen.
Preferably R² represents hydrogen or methyl.
Preferably R³ represents hydrogen, methyl or halogen, more preferably hydrogen. Preferably R^{3a} and R⁵ independently represent hydrogen or methyl.
Preferably m, n and p each represent 1.
When A represents a group -Ar¹, Ar¹ preferably represents optionally substituted phenyl or pyridyl, more preferably phenyl optionally substituted with halogen, cyano, trifluoromethyl or trifluoromethoxy. Particularly preferred Ar¹ is phenyl optionally substituted with halogen (such as 2-fluorine).
When A represents a group -Ar²-Ar³, Ar² and Ar³ preferably both independently represent phenyl or monocyclic heteroaryl group as defined above.
Preferably A represents a group -Ar¹.

### For compounds of formula (I) wherein Q represents a group of formula (ii):

Preferably, R¹ represents hydrogen or methyl, more preferably hydrogen.
Preferably R² represents hydrogen.
Preferably R³ represents hydrogen, methyl or halogen, more preferably hydrogen. Preferably X represents -N=.
Preferably Y represents -N(R⁵)-.
Preferably R^{3a} and R⁵ independently represent hydrogen or methyl, more preferably hydrogen.
Preferably m, n and p each represent 1.
When A represents a group -Ar¹, Ar¹ preferably represents optionally substituted phenyl or pyridyl, more preferably phenyl optionally substituted with halogen, cyano, trifluoromethyl or trifluoromethoxy. Particularly preferred Ar¹ is phenyl optionally substituted with halogen (such as 2-fluorine and 3-fluorine).
When A represents a group -Ar²-Ar³, Ar² and Ar³ preferably both independently represent phenyl or monocyclic heteroaryl group as defined above.
Preferably A represents a group -Ar¹.

### For compounds of formula (I) wherein Q represents a group of formula (iii):

Preferably, R¹ represents hydrogen or methyl, more preferably hydrogen.
Preferably R² represents hydrogen.
Preferably R³ represents hydrogen, methyl or halogen, more preferably hydrogen or methyl.
Preferably R⁴ represents hydrogen or halogen, more preferably hydrogen.

Preferably m, n, p and s each represent 1.
When A represents a group -Ar¹, Ar¹ preferably represents optionally substituted phenyl or pyridyl, more preferably phenyl optionally substituted with halogen, cyano, trifluoromethyl or trifluoromethoxy. Particularly preferred Ar¹ is unsubstituted phenyl. When A represents a group -Ar²-Ar³, Ar² and Ar³ preferably both independently represent phenyl or monocyclic heteroaryl group as defined above;
Preferably A represents a group -Ar¹.

### For compounds of formula (I) wherein Q represents a group of formula (iv):

Preferably, R¹ represents hydrogen or methyl, more preferably hydrogen.
Preferably, R² represents hydrogen.
Preferably, R³ represents hydrogen or a halogen atom.
Preferably, R⁴ represents hydrogen or methyl (particularly 2-methyl).
Preferably, m, p, q and r all represent 1.
When A represents -Ar²Ar³, Ar³ is preferably linked to Ar² via a carbon atom of Ar³, and preferably Ar² and Ar³ independently represent phenyl or a monocyclic heteroaryl group as defined above.
Preferably, A represents -Ar¹.
Preferably, -Ar¹ is phenyl or pyridyl optionally substituted by one or more halogen atoms or cyano, trifluoromethoxy or trifluoromethyl groups, more preferably unsubstituted phenyl, 2-fluorophenyl, 3-fluorophenyl or 3-chlorophenyl.

Preferred compounds according to the invention include examples E1-E16 as shown below, or a pharmaceutically acceptable salt thereof.

The compounds of formula (I) can form acid addition salts thereof. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, **66**, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid. The present invention includes within its scope all possible stoichiometric and non-stoichiometric forms.

The compounds of formula (I) may be prepared in crystalline or non-crystalline form, and, if crystalline, may optionally be solvated, eg. as the hydrate. This invention includes within its scope stoichiometric solvates (eg. hydrates) as well as compounds containing variable amounts of solvent (eg. water).

Certain compounds of formula (I) are capable of existing in stereoisomeric forms (e.g. diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The present invention also provides a process for the preparation of a compound of formula (I) or a pharmaceutically acceptable salt thereof, which process comprises:
(a) oxidation of a compound of formula (II) 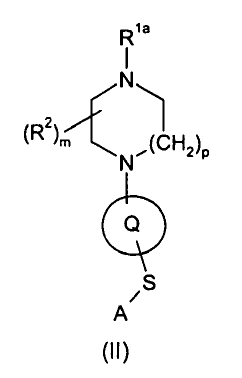 wherein R^{1a} is as defined for R¹ or an *N*-protecting group and Q, R², m, p and A are as defined above, and thereafter as necessary removing an R^{1a} *N*-protecting group; or
(b) preparing a compound of formula (I) wherein Q represents a group of formula (iii) or (iv) which comprises reacting a compound of formula (III) 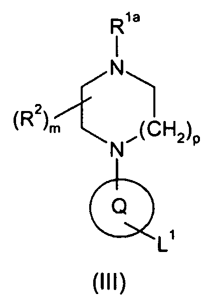 wherein R^{1a} is as defined for R¹ or an *N*-protecting group, R², m and p are as defined above and L¹ is a suitable leaving group such as iodo or trifluoromethanesulfonyloxy; with a compound of formula A-SO₂H, (or A-SH followed by a subsequent oxidation step) wherein A is as defined above and thereafter as necessary removing an R^{1a} *N*-protecting group; or
(c) preparing a compound of formula (I) wherein Q represents a group of formula (iii) or (iv) which comprises reacting a compound of formula (IV) 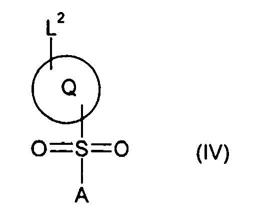 wherein A is as defined above and L² represents a leaving group, such as a halogen atom,
   with a compound of formula (V) 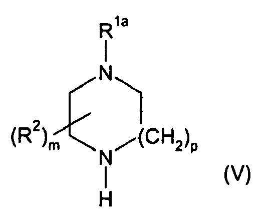 wherein R^{1a} is as defined for R¹ or an *N*-protecting group such as *tert*-butyloxycarbonyl (t-Boc), R², m and p are as defined above, followed by subsequent deprotection as necessary; or
(d) deprotecting a compound of formula (I) which is protected; and thereafter optionally
(e) interconversion to other compounds of formula (I) and/or forming a pharmaceutically acceptable salt and/or solvate.

The *N*-protecting group used may be any conventional group e.g. *t*-butyloxycarbonyl (Boc) or benzyloxycarbonyl.

Process (a) typically comprises the use of an oxidant such as a peracid (e.g. 3-chloroperbenzoic acid or peracetic acid) or potassium monopersulfate, in a suitable solvent such as dichloromethane or aqueous methanol.

Process (b) wherein a compound of formula (III) is reacted with a compound of formula A-SO₂H typically comprises use of basic conditions and may be most conveniently carried out by using a suitable salt of the compound A-SO₂H (e.g. the sodium salt) in an appropriate solvent such as *N*,*N*-dimethylformamide, in the presence of a transition metal salt such as copper (I) iodide.

Process (b) wherein a compound of formula (III) is reacted with a compound of formula A-SH typically comprises use of basic conditions e.g. by using a suitable salt of the compound A-SH (e.g. the sodium salt) in an appropriate solvent such as *N,N-*dimethylformamide, in the presence of a suitable metal salt such as copper (I) iodide, followed by use of a suitable oxidant such as 3-chloroperbenzoic acid, peracetic acid or potassium monopersulfate.

Process (c) typically comprises the use of a palladium catalyst, eg. palladium acetate in the presence of a suitable ligand, eg. BINAP and a suitable base, eg. caesium carbonate in a suitable solvent, eg. dioxane.

In processes (a), (b), (c) and (d), examples of protecting groups and the means for their removal can be found in T. W. Greene 'Protective Groups in Organic Synthesis' (J. Wiley and Sons, 1991). Suitable amine protecting groups include sulphonyl (e.g. tosyl), acyl (e.g. acetyl, 2',2',2'-trichloroethoxycarbonyl, benzyloxycarbonyl or t-butoxycarbonyl) and arylalkyl (e.g. benzyl), which may be removed by hydrolysis (e.g. using an acid such as hydrochloric acid) or reductively (e.g. hydrogenolysis of a benzyl group or reductive removal of a 2',2',2'-trichloroethoxycarbonyl group using zinc in acetic acid) as appropriate. Other suitable amine protecting groups include trifluoroacetyl (-COCF₃) which may be removed by base catalysed hydrolysis, or a solid phase resin bound benzyl group, such as a Merrifield resin bound 2,6-dimethoxybenzyl group (Ellman linker), which may be removed by acid catalysed hydrolysis, for example with trifluoroacetic acid. A further amine protecting group includes methyl which may be removed using standard methods for N-dealkylation (e.g. 1-chloroethyl chloroformate under basic conditions followed by treatment with methanol).

Process (e) may be performed using conventional interconversion procedures such as epimerisation, oxidation, reduction, alkylation, nucleophilic or electrophilic aromatic substitution, ester hydrolysis or amide bond formation. For example, N-dealkylation of a compound of formula (I) wherein R¹ represents an alkyl group to give a compound of formula (I) wherein R¹ represents hydrogen. It will be appreciated that such interconversion may be interconversion of protected derivatives of formula (I) which may subsequently be deprotected following interconversion. It will also be appreciated that attempted conversion of optionally protected compounds of formula (I) wherein R⁵ represents hydrogen into other optionally protected compounds of formula (I) wherein R⁵ represents C₁₋₆alkyl using conventional alkylation methods may give rise to mixtures containing varying amounts of the corresponding regioisomers. Such mixtures may be separated by conventional means, for example using flash chromatography.

Compounds of formula (II) may be prepared by reacting a compound of formula (VI) 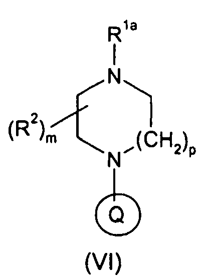 wherein R^{1a} is as defined for R¹ or an *N*-protecting group and R², m and p are as defined above, with a compound of formula A-S-L³ or A-S-S-A, wherein A is as defined above and L³ is a leaving group such as halogen or methylsulfonyl. When Q represents a group of formula (i) or (ii) this reaction typically comprises the use of a base, for example in the case where X represents -N=, Y represents -N(R⁵)- and R⁵ represents hydrogen, a metal hydride (eg. sodium hydride) in a suitable solvent such as *N,N-*dimethylformamide which is then allowed to react with the compound of formula A-S-L³ or A-S-S-A.

Compounds of formula (VI) wherein Q represents a group of formula (i), X represents - N=, Y represents -N(R⁵)- and R⁵ represents hydrogen may be prepared in accordance with the following process: 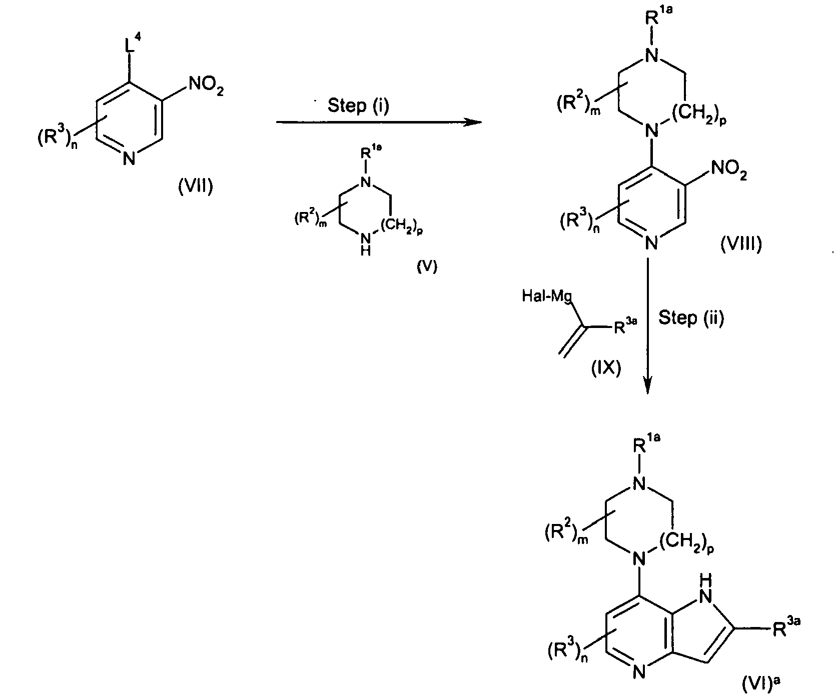 wherein R^{1a} is as defined for R¹ or an *N*-protecting group and R², R³, R^{3a}, m, n and p are as defined above, L⁴ represents a suitable leaving group such as halogen (eg. chlorine), Hal is a halogen atom such as chlorine or bromine.

Step (i) typically comprises the use of a base such as triethylamine or an excess of the compound of formula (V) and an inert solvent such as dichloromethane.

Step (ii) typically comprises the use of an inert solvent such as tetrahydrofuran at a suitable temperature (e.g. -40 °C).

Compounds of formula (VI) wherein Q represents a group of formula (ii), X represents - N=, Y represents -N(R⁵)- and R⁵ represents hydrogen may be prepared in an analogous manner to that described for the process above.

Compounds of formula (III) may be prepared in accordance with the following process: 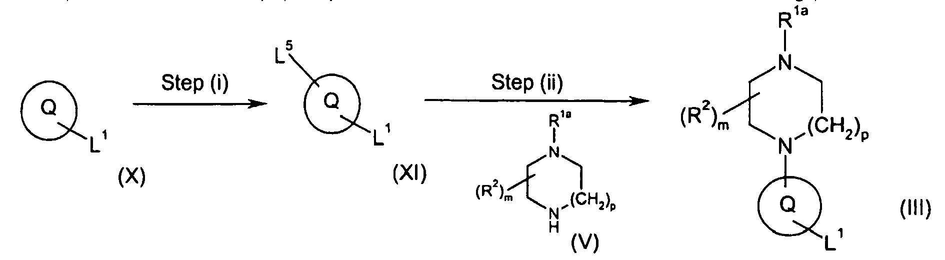 wherein R^{1a} is as defined above for R¹ or an *N*-protecting group, R², m and p are as defined above, Q represents a group of formula (iii) or (iv), L¹ represents a suitable leaving group, such as iodo or trifluoromethylsulfonyloxy and L⁵ represents a suitable leaving group, such as chlorine.

Step (i) typically comprises reacting a compound of formula (X) with a suitable oxidant such as a peracid (e.g. 3-chloroperbenzoic acid or peracetic acid) in an inert solvent such as dichloromethane in order to generate the quinoline-N-oxide, followed by a combination of Lewis acid and nucleophile; for example this latter step may be advantageously carried out using phosphorus oxychloride.

Step (ii) typically comprises heating a mixture of compounds of formula (XI) and (V) in a suitable solvent such as ethanol, optionally in the presence of additional base (e.g. triethylamine or an excess of the compound of formula (V)).

Compounds of formula (IV) wherein Q represents a group of formula (iv) may be prepared in accordance with the following process: 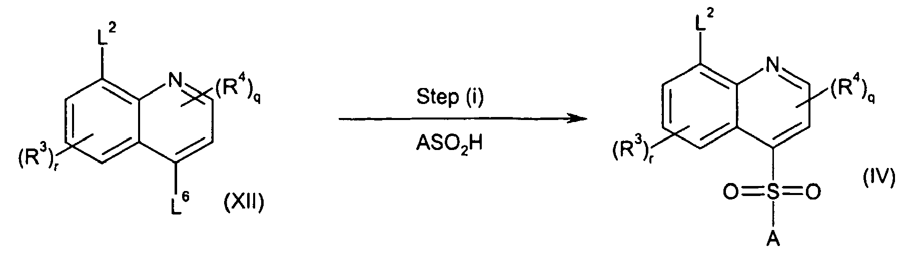 wherein R³, R⁴, r, q, A and L² are as defined above and L⁶ represents a suitable leaving group, such as halogen atom.

Step (i) is typically carried out under basic conditions, for example using the sodium salt of the sulfinic acid compound in the presence of a suitable solvent, eg. dimethylformamide. Alternatively this transformation may be carried out using a compound of formula A-SH and subsequent oxidation in a manner similar to that described above for process (b).

Compounds of formula (III) wherein Q represents a group of formula (iv) and L¹ represents a halogen atom (Hal) may be prepared in accordance with the following process: 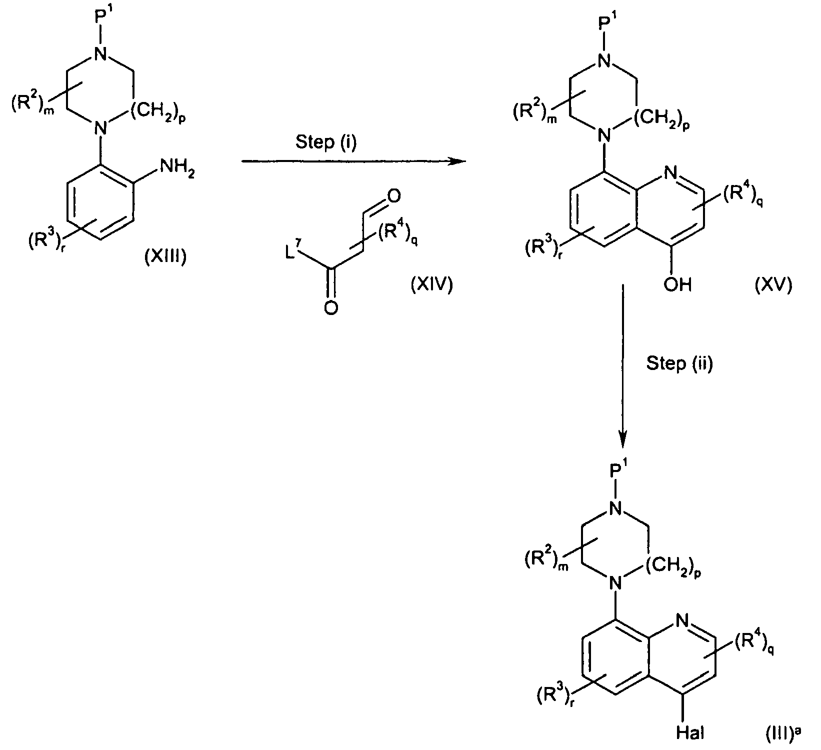 wherein R², R³, R⁴, m, p, r and q are as defined above, L⁷ represents a suitable leaving group, eg. an alkoxy group and P¹ represents a suitable protecting group, eg. trifluoroacetyl.

Step (i) typically comprises an initial condensation reaction under acidic conditions, followed by a thermal cyclisation of the resulting enamine in the presence of a suitably high boiling solvent, eg. diphenyl ether.

Step (ii) typically comprises the use of a suitable phosphorus halide or phosphoryl halide at an elevated temperature.

Compounds of formula (V), (VII), (IX), (X), (XII), (XIII) and (XIV) are known in the literature or can be prepared by analogous methods.

Pharmaceutically acceptable salts may be prepared conventionally by reaction with the appropriate acid or acid derivative.

Compounds of formula (I) and their pharmaceutically acceptable salts have affinity for the 5-HT₆ receptor and are believed to be of potential use in the treatment of certain CNS disorders such as anxiety, depression, epilepsy, obsessive compulsive disorders, migraine, cognitive memory disorders (e.g. Alzheimers disease, age related cognitive decline and mild cognitive impairment), Parkinsons Disease, ADHD (Attention Deficit Disorder/Hyperactivity Syndrome), sleep disorders (including disturbances of Circadian rhythm), feeding disorders such as anorexia and bulimia, panic attacks, withdrawal from drug abuse such as cocaine, ethanol, nicotine and benzodiazepines, schizophrenia (in particular cognitive deficits of schizophrenia), stroke and also disorders associated with spinal trauma and/or head injury such as hydrocephalus. Compounds of the invention are also expected to be of use in the treatment of certain GI (gastrointestinal) disorders such as IBS (Irritable Bowel Syndrome). Compounds of the invention are also expected to be of use in the treatment of obesity.

Thus the invention also provides a compound of formula (I) or a pharmaceutically acceptable salt thereof, for use as a therapeutic substance, in particular in the treatment or prophylaxis of the above disorders. In particular the invention provides for a compound of formula (I) or a pharmaceutically acceptable salt thereof, for use in the treatment of depression, anxiety, obesity and cognitive memory disorders

In another aspect, the invention provides the use of a compound of formula (I) or a pharmaceutically acceptable salt thereof in the manufacture of a medicament for use in the treatment or prophylaxis of the above disorders.

5-HT₆ antagonists have the potential to be capable of increasing basal and learning-induced polysialylated neuron cell frequency in brain regions such as the rat medial temporal lobe and associated hippocampus, as described in International Patent Application No. PCT/EP03/00462. Thus, according to a further aspect of the present invention, we provide a method of promoting neuronal growth within the central nervous system of a mammal which comprises the step of administering a compound of formula (I) or a pharmaceutically acceptable salt thereof.

In order to use the compounds of formula (I) in therapy, they will normally be formulated into a pharmaceutical composition in accordance with standard pharmaceutical practice. The present invention also provides a pharmaceutical composition, which comprises a compound of formula (I) or a pharmaceutically acceptable salt thereof, and a pharmaceutically acceptable carrier.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusable solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1 % to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 0.05 to 200 mg, for example 20 to 40 mg; and such unit doses will preferably be administered once a day, although administration more than once a day may be required; and such therapy may extend for a number of weeks or months.

The following Descriptions and Examples illustrate the preparation of compounds of the invention.

### Description 1

### 4-(4-tert-Butyloxycarbonyl)piperazin-1-yl-3-nitropyridine (D1)

To a stirred suspension of 4-chloro-3-nitropyridine [Carceller *et al., J. Med. Chem.* **1996** 39 487] (23.37 g, 0.147 mol) in dichloromethane (500 ml) under argon was added Et₃N (22.43 ml), followed by 1-Boc-piperazine (30.38 g, 0.14 mol). The reaction was left to stir for 72 h at room temperature. The solvent was then evaporated *in vacuo* and the residues partitioned between dichloromethane (250 ml) and water (250 ml). The organic layer was then washed with 10% citric acid (250 ml), sat. NaHCO₃ (250 ml), brine (250 ml), dried (MgSO₄) and the solvents evaporated *in vacuo* to give the product as a dark yellow solid (D1) (45 g)
NMR (DMSO-d₆) : δ_{H} 1.42 (9H, s), 3.24-3.26 (4H, m), 3.46-3.47 (4H, m), 7.17-7.18 (1H, d), 8.38-8.39 (1H, d), 8.79 (1H, s)

### Description 2

### 7-(4-tert-Butyloxycarbonyl)piperazin-1-yl-1H-pyrrolo[3,2-b]pyridine (D2)

To a solution of 4-(4-*tert*-butyloxycarbonyl)piperazin-1-yl-3-nitropyridine (D1) (6.3 g, 20.5 mmol) in tetrahydrofuran (200 ml) at -50 °C under argon was added vinyl magnesium bromide (1M in THF; 67.4ml, 67.4 mmol) was added rapidly, keeping the temperature below - 40 °C. Reaction stirred at - 40 °C for 30 min, then poured into sat. NH₄Cl (1000 ml) and extracted with dichloromethane (2 x 500 ml). The combined organic layers were then dried (MgSO₄) and the solvent evaporated *in vacuo.* Purification by flash chromatography (MeOH / dichloromethane) gave the product as a brown solid (D2) (2.3 g).
NMR (CDCl₃) : δ_{H} 1.49 (9H, s), 3.35-3.39 (4H, m), 3.64-3.68 (4H, m), 6.59-6.61 (1H, d), 6.66-6.67 (1H, d), 7.39-7.41 (1H, d), 8.25-8.27 (1H, d), 11.45 (1H, br s)
Mass Spectrum: C₁₆H₂₂N₄O₂ requires 302; found: 303 (MH⁺)

### Description 3

### 3-Phenylsulfanyl-7-(4-tert-butyloxycarbonyl)piperazin-1-yl-1H-pyrrolo[3,2-b] pyridine (D3)

Sodium hydride (60% in mineral oil, 39.7 mg, 0.99 mmol) was washed in hexane and then taken up in dimethylformamide (4 ml). 7-(4-*tert*-Butyloxycarbonyl)piperazin-1-yl-1*H-*pyrrolo[3,2-*b*]pyridine (D2) (200 mg, 0.66 mmol) was added and left to stir for 10min. Diphenyldisulfide (159 mg, 0.73 mmol) was added and the reaction left to stir for 16 h. The reaction mixture was then diluted with water (10 ml) and extracted with ethyl acetate (3 x 20 ml). The combined organic extracts were washed with water (50 ml), dried (MgSO₄) and the solvents evaporated *in vacuo.* Purification by flash chromatography (MeOH : DCM) gave the product as an off-white solid (D3) (115.3 mg).
NMR (CDCl₃): δ_{H} 1.49 (9H, s), 3.35-3.39 (4H, m), 3.64-3.68 (4H, m), 6.58-6.61 (1H, d), 6.99-7.12 (5H, m), 7.63 (1H, s), 8.22-8.24 (1H, d), 12.45 (1H, br s).
Mass Spectrum: C₂₂H₂₆N₄O₂S requires 410; found: 411 (MH⁺)

### Description 4

### 3-Phenylsulfonyl-7-(4-tert-butyloxycarbonyl)piperazin-1-yl-1H-pyrrolo[3,2-b] pyridine (D4)

To a stirred solution of 3-phenylsulfanyl-7-(4-*tert*-butyloxycarbonyl)piperazin-1-yl-1*H-*pyrrolo[3,2-*b*] pyridine (D3) (50 mg, 0.12 mmol) in methanol (5 ml) was added potassium monopersulfate (150 mg, 0.24 mmol) in water (1 ml). The reaction mixture was stirred for 90 min and the solvents evaporated *in vacuo.* The residue was partitioned between dichloromethane (10 ml) and sat. NaHCO₃ solution (10 ml). The aqueous layer was re-extracted with dichloromethane and the combined organic extracts dried (MgSO₄) and the solvents evaporated *in* vacuo to give the product as a white solid (D4) (54.2 mg). NMR (CDCl₃) : δ_{H} 1.47 (9H, s), 3.67-3.70 (4H, m), 3.90-4.30 (4H, br m), 6.46-6.48 (1H, d), 7.44-7.52 (3H, m), 7.85 (1H, br s), 7.95-8.00 (3H, m), 10.50 (1H, br s)
Mass Spectrum: C₂₂H₂₆N₄O₄S requires 442; found: 441 (M-H)

### Descriptions 5 and 6

### 7-(4-tert-butyloxycarbonyl)piperazin-1-yl-4-methyl-3-phenylsulfonyl-4H-pyrrolo[3,2-b] pyridine (D5) and 7-(4-tert-butyloxycarbonyl)piperazin-1-yl-1-methyl-3-phenylsulfonyl-1H-pyrrolo[3,2-b]pyridine (D6)

To a stirred solution of 3-phenylsulfonyl-7-(4-*tert*-butyloxycarbonyl)piperazin-1-yl-1*H-*pyrrolo[3,2-b] pyridine (D4) (52.7 mg, 0.12 mmol) in ethanol (4 ml) was added potassium hydroxide (8.5 mg, 0.18 mmol). After 20min, the solvent was evaporated *in vacuo* and the residue re-dissolved in acetone (2 ml). Dimethyl sulfate (15 mg, 0.12 mmol) was then added and reaction stirred for 2 h. The reaction mixture was diluted with dichloromethane (10 ml) and washed with water (10 ml), dried (MgSO₄) and the solvents evaporated *in vacuo.* Purification by flash chromatography (EtOAc : dichloromethane) gave 7-(4-*tert-*butyloxycarbonyl) piperazin-1-yl-4-methyl-3-phenylsulfonyl-4*H*-pyrrolo[3,2-*b*] pyridine (D5) (8.8 mg) and 7-(4-*tert*-butyloxycarbonyl)piperazin-1-yl-1-methyl-3-phenylsulfonyl-1*H*-pyrrolo[3,2-*b*] pyridine (D6) (26 mg) as brown oils.
7-(4-*tert*-butyloxycarbonyl) piperazin-1-yl-4-methyl-3-phenylsulfonyl-4*H*-pyrrolo [3,2-*b*] pyridine (D5):
NMR (CDCl₃) : δ_{H} 1.49 (9H, s), 3.00 (4H, br s), 3.82 (4H, br s), 4.11 (3H,s), 6.28-6.30 (1H, d), 7.29 (1H, s), 7.44-7.51 (3H, m), 7.87-7.89 (2H, m), 8.17 (1H, s)
Mass Spectrum: C₂₃H₂₈N₄O₄S requires 456; found: 457 (MH⁺)
7-(4-*tert*-butyloxycarbonyl)piperazin-1-yl-1-methyl-3-phenylsulfonyl-1*H*-pyrrolo [3,2-*b*] pyridine (D6):
NMR (CDCl₃): δ_{H} 1.49 (9H, s), 3.00 (4H, br s), 3.50 (4H, br s), 4.11 (3H,s), 6.82-6.83 (1H, d), 7.44-7.51 (3H, m), 7.84 (1H, s), 8.27-8.29 (2H, m), 8.49-8.51 (1H, d)
Mass Spectrum: C₂₃H₂₈N₄O₄S requires 456; found 457 (MH⁺)

### Description 7

### 2-(4-tert-Butyloxycarbonyl)piperazin-1-yl-3-nitro pyridine (D7)

To a stirred solution of 2-chloro-3-nitro pyridine (6.5 g, 0.041 mol) in dichloromethane was added 1-Boc-piperazine (8.0 g, 0.043 mol) and triethylamine (6.29 ml, 0.045 mol). The reaction was then stirred at room temperature, under argon for 3 h. The solvents were evaporated *in vacuo* and the residue partitioned between dichloromethane (150 ml) and water (100 ml). Aqueous layer was re-extracted with dichloromethane (2 x 150 ml) and the combined organic layers washed with 10% citric acid (100 ml), saturated aqueous NaHCO₃ (100 ml), water (100 ml), dried (Na₂SO₄) and the solvents evaporated *in vacuo* to give a yellow oil (D7) (12.1 g)
¹H NMR (CDCl₃) : δ 1.48 (9H, s), 3.40-3.50 (4H, m), 3.55-3.58 (4H, m), 6.78-6.81 (1H, m), 8.15 (1H, dd, J = 1.7, 8.0 Hz), 8.35 (1H, m)
Mass Spectrum: C₁₄H₂₀N₄O₄ requires 308; Found 209 ((M-Boc)H⁺)

### Description 8

### 7-(4-tert-Butyloxycarbonyl)piperazin-1-yl-1H-pyrrolo[2,3-c]pyridine (D8)

To a stirred solution of 2-(4-*tert*-butyloxycarbonyl)piperazin-1-yl-3-nitro pyridine (D7) (1.0 g, 3.25 mmol) in THF at -50 °C, under argon was added vinyl magnesium bromide (1.0 M in THF; 10.6 ml, 11.0 mmol) in one portion. Reaction was stirred at -40 to -45 °C for 30 min before being quenched with sat. NH₄Cl solution. The mixture was extracted with dichloromethane (2 x 100 ml) and the combined organic layers dried (Na₂SO₄) and the solvent evaporated *in vacuo* to give an orange oil. Purification by flash chromatography (dichloromethane / MeOH; 98:2 - 95:5), followed by re-purification (dichloromethane / EtOAc 95:5) gave the product as an orange solid (D8) (86 mg)
¹H NMR (CDCl₃) : δ 1.49 (9H, s), 3.35-3.40 (4H, m), 3.62-3.66 (4H, m), 6.54 (1H, dd, J = 2.0, 3.0 Hz), 7.22 (1H, d, J = 5.6 Hz), 7.28 (1H, dd, J = 2.8, 3.0 Hz) 7.92 (1H, d, J = 5.6 Hz), 8.45 (1H, br s)
Mass Spectrum: C₁₆H₂₂N₄O₂ requires 302; Found 303 (MH⁺)

### Description 9

### 7-(4-tert-Butyloxycarbonyl)piperazin-1-yl-3-phenylsulfanyl-1H-pyrrolo[2,3-c] pyridine (D9)

Sodium hydride (60% in oil; 26 mg, 0.65 mmol) was washed with dry Et₂O and then dried under argon. This was then slurried in dry DMF (1 ml) and 7-(4-*tert-*butyloxycarbonyl)piperazin-1-yl-1*H*-pyrrolo[2,3-*c*]pyridine (D8) (130 mg, 0.43 mmol) in DMF (3 ml) was added, under argon. Reaction mixture was stirred at room temperature for 10 min, then diphenyldisulfide (103 mg, 0.47 mmol) was added and stirring continued at room temperature for 18 h. After this period, reaction was quenched with water (40 ml) and the mixture extracted with EtOAc (3 x 50 ml). Combined organic layers were washed with water, dried (Na₂SO₄) and the solvent evaporated *in vacuo* to give a red oil. Purification by flash chromatography (EtOAc / dichloromethane 20:80) gave the product as a pale red solid (D9) (85 mg)
NMR (CDCl₃) : δ_{H} 1.50 (9H, s), 3.39-3.43 (4H, m), 3.65-3.67 (4H, m), 7.08-7.10 (3H, m), 7.15-7.18 (3H, m), 7.53 (1H, d, *J* = 2.7 Hz) 7.95 (1H, d, *J* = 5.6 Hz), 8.55 (1H, br s)
Mass Spectrum: C₂₂H₂₆N₄O₂S requires 410; Found 411 (MH⁺)

### Description 10

### 7-(4-tert-Butyloxycarbonyl)piperazin-1-yl-3-phenylsulfonyl-1H-pyrrolo[2,3-c] pyridine (D10)

To a stirred solution of 7-(4-*tert*-butyloxycarbonyl)piperazin-1-yl-3-phenyl sulfanyl-1*H-*pyrrolo[2,3-c] pyridine (D9) (57 mg, 0.139 mmol) in MeOH (5 ml) was added potassium monopersulfate (85 mg, 0.139 mmol) dissolved in water (1 ml). Reaction mixture was stirred at room temperature for 18 h before a further 1 eq. of potassium monopersulfate was added and reaction stirred for an additional 1 h. The solvents were evaporated in *vacuo* and the residue partitioned between dichloromethane and sat. NaHCO₃ solution. The aqueous layer re-extracted with dichloromethane (3 x 50 ml) and the combined organic layers washed with water (50 ml), dried (Na₂SO₄) and the solvents evaporated in *vacuo* to give a brown solid. This material was then dissolved in triethylphosphite (3 ml) and heated at 130 °C for 3 h. After this period, reaction mixture cooled to room temperature and diluted with dichloromethane (50 ml), washed with water (2 x 50 ml) and the combined aqueous layers re-extracted with dichloromethane (2 x 50 ml). The combined organic layers were dried (Na₂SO₄) and the solvents evaporated *in vacuo.* Purification by flash chromatography (EtOAc / dichloromethane 2:98 - 20:80) gave the product as a white solid (D10) (55 mg)
NMR (CDCl₃) : δ_{H} 1.49 (9H, s), 3.33-3.36 (4H, m), 3.61-3.63 (4H, m), 7.46-7.51 (4H, m), 7.93 (1H, d, J = 3 Hz), 8.01-8.05 (3H, m), 8.85 (1H, br s)
Mass Spectrum: C₂₂H₂₆N₄O₄S requires 442; Found 443 (MH⁺)

### Description 11

### 7-Iodo-2-methylquinoline (D11)

Crotonaldehyde (4.96 ml, 60.0 mmol) was added dropwise over 1 h, *via* syringe pump, to a solution of 3-iodoaniline (12.5 g, 57.0 mmol) in 5M HCl (30 ml) at 90 °C. The reaction mixture was then heated at 100 °C for 3 h, before being cooled to room temperature and washed with Et₂O. To the aqueous solution was then added approx. 1 eq. of zinc (II) chloride, with vigorous stirring. The solution was then cooled to 0 °C and stirred for 45 min. The solid material was filtered off and washed (x 2) with cold 5M HCl. The crude product was dried on a filter paper and then stirred as a suspension in 2-propanol. The zinc chloride salt was filtered, dried and then re-suspended in water. The stirred mixture was basified with conc. ammonium hydroxide and the resultant slurry extracted with EtOAc (3 x 100 ml). The combined organic layers were dried (Na₂SO₄) and the solvents evaporated *in vacuo* to give the free base as a dark green oil. This material was then dissolved in MeOH and 1.1 eq. of 1 M HCl in Et₂O added. The solvents were then evaporated *in vacuo* to give a dark green solid. Re-crystallisation from MeOH gave the hydrochloride salt as a green solid. The free base was regenerated by stirring a suspension of the salt in saturated aqueous NaHCO₃ and extraction with dichloromethane to give the title compound (D11) (3.05 g).
NMR (CDCl₃) : δ_{H} 2.74 (3H, s), 7.30 (1H, d, J = 8.4 Hz), 7.49 (1H, d, J = 8.5 Hz), 7.74 (1H, dd, J = 1.6, 8.5 Hz), 8.00 (1H, d, J = 8.4 Hz), 8.46 (1H, s)
Mass Spectrum : C₁₀H₈IN requires 269; found: 270 (MH⁺).

### Description 12

### 4-Chloro-7-iodo-2-methylquinoline (D12)

To a solution of 7-iodo-2-methylquinoline (D11) (1.6 g, 5.95 mmol) in chloroform (30 ml) was added 3-chloroperbenzoic acid (- 50 %, 2.46 g, 7.14 mmol) in one portion. The reaction mixture was stirred at room temperature for 1 h, diluted with dichloromethane (100 ml) and then washed with saturated aqueous NaHCO₃. The aqueous layer was re-extracted with dichloromethane and the combined organic layers dried (Na₂SO₄) and the solvents evaporated *in vacuo* to give 7-iodo-2-methylquinoline-N-oxide as an orange oil which was used without further purification. To a stirred solution of the 7-iodo-2-methylquinoline-*N*-oxide (1.70 g, 5.96 mmol) in dichloromethane (50 ml) was added POCl₃ (0.61 ml, 6.56 mmol) at 0 °C, under argon. Reaction mixture was stirred at room temperature for 12 h before a further 1 eq. of POCl₃ was added. Reaction mixture was then heated at 50 °C for 1.5 h, a further 1 eq. of POCl₃ added and the reaction heated at 60 °C for 2 h. After this period, the reaction mixture was cooled to room temperature and poured into ice / water (300 ml), basified with 0.88 NH₃ and extracted with dichloromethane (3 x 100 ml). Combined organic layers were washed with water (100 ml), dried (Na₂SO₄) and solvents evaporated *in vacuo* to give a brown oil. Purification by flash chromatography (5% EtOAc in PE) gave the title compound as a yellow solid (D12) (485 mg).
NMR (CDCl₃) : δ_{H} 2.71 (3H, s), 7.40 (1 H, s), 7.83 (1H, dd, *J* = 1.6, 8.8 Hz), 7.88 (1H, d, *J* = 8.8 Hz), 8.46 (1H, d, J = 1.6 Hz)
Mass Spectrum : C₁₀H₇^{35/37}CIIN requires 303 / 305; Found 304 / 306 (MH⁺)

### Description 13

### 4-(4-tert-Butyloxycarbonyl)piperazin-1-yl-7-iodo-2-methylquinoline (D13)

A mixture of 4-chloro-7-iodo-2-methylquinoline (D12) (475 mg, 1.56 mmol) and 1-Boc-piperazine (348 mg, 1.88 mmol) in ethanol (2 ml) were heated at 130 °C for 4 h in a sealed vessel. The reaction mixture was then cooled, the solvent evaporated *in vacuo* and the residue partitioned between dichloromethane and saturated aqueous NaHCO₃. The aqueous layer was re-extracted with dichloromethane (x 2) and the combined organic layers dried (Na₂SO₄) and the solvent evaporated *in vacuo* to give a yellow oil. Purification by flash chromatography (EtOAc / PE) gave the title compound as a yellow solid (D13) (485 mg).
NMR (CDCl₃) : δ_{H} 1.50 (9H, s), 2.66 (3H, s), 3.12-3.14 (4H, m), 3.68-3.71 (4H, m), 6.74 (1H, s), 7.66-7.68 (2H, m), 8.40 (1H, d, J = 1.6 Hz)
Mass Spectrum : C₁₉H₂₄ IN₃O₂ requires 453; Found 454 (MH⁺)

### Description 14

### 4-(4-tert-Butyloxycarbonyl)piperazin-1-yl-2-methyl-7-phenylsulfonylquinoline (D14)

A mixture of 4-(4-*tert*-butyloxycarbonyl)piperazin-1-yl-7-iodo-2-methylquinoline (D13) (100 mg, 0.22 mmol), phenylsulfinic acid sodium salt (132 mg, 0.66 mmol) and Cul (126 mg, 0.66 mmol) were stirred together under argon, excluding light for 20 min. DMF (5 ml) was then added and the reaction heated at 120 °C for 24 h. the reaction mixture was then cooled to room temperature and partitioned between water (60 ml) and dichloromethane (60 ml). Aqueous layer was re-extracted with dichloromethane and the combined organic layers washed with water, dried (Na₂SO₄) and the solvents evaporated *in vacuo* to give a yellow oil. Purification by flash chromatography (EtOAc / PE) gave the title compound as an off-white solid (D14) (65 mg)
NMR (CDCl₃) : δ_{H} 1.50 (9H, s), 2.69 (3H, s), 3.11-3.14 (4H, m), 3.68-3.71 (4H, m), 6.81 (1H, s), 7.48-7.56 (3H, m), 7.89 (1H, d, *J* = 8 Hz), 8.00-8.07 (3H, m), 8.59 (1H, d, *J* = 2 Hz)
Mass Spectrum : C₂₅H₂₉N₃O₄S requires 467; Found 468 (MH⁺)

### Description 15

### 8-Iodo-4-phenylsulfonylquinoline (D15)

Phenylsulfinic acid sodium salt (621 mg, 2.7 mmol), dissolved in DMF (10 ml), was added to a stirred solution of 4-bromo-8-iodoquinoline (300 mg, 0.89 mmol) in DMF (10 ml) at room temperature under argon. Reaction then stirred at 100 °C for 16 h. Reaction mixture was then diluted with water (50 ml) and extracted with EtOAc (3 x 50 ml). Combined organic layers were washed with brine (50 ml), dried (MgSO₄) and the solvents evaporated *in vacuo.* Purification by flash chromatography (EtOAc / hexane) gave the title product as a yellow solid (306 mg).
¹H NMR (CDCl₃): δ 7.32-7.39 (1H, t), 7.49-7.65 (3H, m), 7.96-8.00 (2H, m), 8.21-8.23 (1H, d), 8.40-8.44 (1H, dd), 8.67-8.71(1H, dd), 9.19-9.21(1H, d).
MS : C₁₅H₁₀INSO₂ requires 395; found 396 (MH⁺)

### Description 16

### 8-(4-tert-Butyloxycarbonyl-piperazin-1-yl)-4-phenylsulphonyl quinoline (D16)

2,2'-Bis(diphenylphosphino)-1,1'-binaphthyl (BINAP) (48 mg, 0.08 mmol), Pd(OAc)₂ (11.4 mg, 0.05 mmol) and Cs₂CO₃ (248 mg, 0.75 mmol) were combined in dioxane (5 ml) under argon and sonicated for 45 min. 1-Boc piperazine (236 mg, 1.28 mmol) and 8-iodo-4-phenylsulfonylquinoline (D15) (200 mg, 0.51 mmol) were combined in dioxane (5 mL) and added to the resulting blood red solution. Reaction was then left to heat at 100 °C for 16 h. The solvent was then evaporated *in vacuo* and the residue partitioned between DCM (10 ml) and water (10 ml). The organic layer was removed and the aqueous layer re-extracted with DCM (10 ml). Combined organics were washed with sat. NaHCO₃ (20 ml), 10% citric acid solution (20 mL), brine (20 mL), dried (MgSO₄) and the solvents evaporated *in vacuo.* Purification by Flash chromatography (EtOAc/hexane) gave the title product as a yellow oil (51.2 mg).
¹H NMR (CDCl₃) : δ 1.49 (9H, s), 3.27-3.31 (4H, m), 3-71-3.75 (4H, m), 7.14-7.15 (1H, dd), 7.48-7.59 (4H, m), 7.96-7.99 (2H, m), 8.17-8.19 (1H, d), 8.22-8.25 (1H, dd), 9.07-9.09 (1H, d).
MS : C₂₄H₂₇N₃SO₄ requires 453; found 454 (MH⁺)

### Description 17

### 2-(4-Methylpiperazin-1-yl) nitrobenzene (D17)

1-Fluoro-nitrobenzene (17.7 ml, 0.168 mol), 1-methyl-piperazine (16 g, 0.16 mol) and K₂CO₃ (24.3 g, 0.176 mol) were combined in DMSO (140 ml) and heated to 140 °C for 16 h. The reaction mixture was then cooled and partitioned between water (300 ml) and EtOAc (300ml). The aqueous was re-extracted with EtOAc (300 ml) and the combined organics washed with water (600 ml), dried (MgSO₄) and the solvents evaporated *in vacuo* to give the title product as a dark orange oil (35.35 g)
¹H NMR (CDCl₃) : δ 2.36 (3H,s), 2.56-2.62 (4H, m), 3.08-3.10 (4H, m), 7.02-7.05 (1H, t), 7.14-7.16 (1H, dd), 7.45-7.50 (1H, m), 7.74-7.77 (1H, dd)

### Description 18

### 2-(4-Trifluoroacetyl-piperazin-1-yl) nitrobenzene (D18)

α-chloroethylchloroformate (7.7 ml) was added to a solution of 2-(4-Methyl piperazin-1-yl) nitrobenzene (D17) (10 g, 45.2 mmol) in DCM (150 ml) with rapid stirring. Diisopropylethylamine (DIPEA) (12.4 ml) was then added and the solution refluxed for 1 h. The solvent was then evaporated *in* vacuo and the residue refluxed in MeOH (150 ml) for 1 h. The solvent was then evaporated *in vacuo,* and the residue taken up in DCM (150ml), under argon. Solution then cooled in an ice-bath and 2,6-lutidine (12.2 ml) added. Trifluoroacetic anhydride (6 ml) in DCM (50 ml) was then added dropwise and the solution left to stir for 16 h. The solution was washed with 10% citric acid solution (2 x 200 ml), brine (200 ml), dried (MgSO₄) and solvents evaporated *in vacuo.* Purification by flash chromatography (EtOAc/hexane) gave the title product as an orange solid (3.48 g).
¹H NMR (CDCl₃) : δ 3.11-3.15 (4H, m), 3.77-3.79 (2H, m), 3.85-3.88 (2H, m), 7.17-7.21 (2H, m), 7.53-7.57 (1H, m), 7.82-7.84 (1H, dd)
MS : C₁₂H₁₂F₃N₃O₂ requires 303; found 304 (MH⁺)

### Description 19

### 2-(4-Trifluoroacetyl-piperazin-1-yl)aniline (D19)

2-(4-trifluoroacetyl-piperazin-1-yl) nitrobenzene (D18) (3.34 g, 11 mmol) was dissolved in EtOH (150 ml) under argon and palladium (10 % Pd on C paste, 300 mg) was added. Reaction mixture was hydrogenated at 1 atm for 16 h. The solution was then filtered through celite and concentrated to yield the title product as an off-white solid (2.99 g).
¹H NMR (DMSO) : δ 2.84-2.88 (4H, bs), 3.34 (4H, bs), 5.11 (2H, bs), 6.54-6.58 (1H, m), 6.69-6.71 (1H, m), 6.82-6.92 (2H, m)
MS : C₁₂H₁₄F₃N₃O requires 273; Found 274 (MH⁺)

### Description 20

### 3-{2-[4-Trifluoroacetyl-piperazin-1-yl]-phenylamino}-but-2-enoic acid ethyl ester (D20)

2-(4-trifluoroacetyl-piperazin-1-yl) aniline (D19) (0.77 g, 2.7 mmol), ethylacetoacetate (0.36 g, 2.7 mmol) and acetic acid (0.17 ml) were stirred in toluene (5mL) and then refluxed in Dean-Stark apparatus. The solvent was evaporated *in vacuo* and the residue purified by flash chromatography (EtOAc/hexane) to yield the title product as a clear oil (0.12 g)
¹H NMR (CDCl₃) : δ 1.25-1.30 (3H, t), 2.12 (3H, s), 2.94-2.97 (4H, m), 3.80-3.82 (2H, m), 3.89-3.91 (2H, m), 4.13-4.18 (2H, q), 4.73 (1H, s), 6.99-7.14 (4H, m), 10.70 (1H, s)
MS : C₁₈H₂₂F₃N₃O₃ requires 385; Found 386 (MH⁺)

### Description 21

### 1,4-Dihydro-2-methyl-8-(4-trifluoroacetylpiperazin-1-yl)-1H-quinoline-4-one (D21)

(3-{2-[4-(Trifluoroacetyl)-piperazin-1-yl]-phenylamino}-but-2-enoic acid ethyl ester (D20) (117 mg, 0.30 mmol) was refluxed in diphenyl ether (1 ml) for 30 min. The solution was then eluted through a Sep-Pak column (EtOAc/hexane, then MeOH) to give the title product as a brown oil (84 mg).
¹H NMR (CDCl₃) : δ 2.44 (3H, s), 3.08 (4H, bs), 3.20 (1H, bs), 3.65 (1H, bs), 4.13 (1H, bs), 4.63 (1H, bs), 6.13-6.14 (1H, d), 7.24-7.31 (1H, m), 7.40-7.43 (1H, dd), 8.13-8.16 (1H, dd), 9.08 (1H, bs)
MS : C₁₆H₁₆F₃N₃O₂ requires 339; Found 340 (MH⁺)

### Description 22

### 4-Chloro-2-methyl-8-(4-trifluoroacetyl-piperazin-1-yl)-quinoline (D22)

1,4-Dihydro-2-methyl-8-(4-trifluoroacetylpiperazin-1-yl)-1*H*-quinoline-4-one (D21) (84 mg, 0.25 mmol) was refluxed in POCl₃ (2 ml) for 2 h. The solution was then diluted with water (5 ml) and basified with 2*M* NH₄OH. Reaction mixture was then extracted with DCM (2 x 10ml) and the combined organic layers washed with water (20 ml), dried (MgSO₄), and the solvent evaporated *in vacuo* to give the title product as a brown oil (88 mg).
¹H NMR (COCl₃) : δ 2.73 (3H, s), 3.42-3.49 (4H, m), 3.93-3.97 (2H, m), 4.01-4.05 (2H, m), 7.13-7.16 (1H, d), 7.41 (1H, s), 7.45-7.52 (1H, t), 7.87-7.91 (1H, dd)
MS : C₁₆H₁₅ClF₃N₃O requires 357; Found 358 (MH⁺)

### Description 23

### 2-Methyl-4-phenylsulfonyl-8-(4-trifluoroacetyl-piperazin-1-yl) quinoline (D23)

4-Chloro-2-methyl-8-(4-trifluoroacetyl-piperazin-1-yl)-quinoline (D22) (88 mg, 0.25 mmol) was dissolved in DMF (5 ml) under argon. Phenylsufinate sodium salt in DMF (5 ml) was added and the solution heated to 100°C for 16h. Reaction mixture was then diluted with water (10 ml) and extracted with EtOAc (2 x 10 ml). The combined organic layers were washed with brine (20 ml), dried (MgSO₄) and the solvents evaporated *in vacuo* to give the title product as a brown oil (106.2 mg).
¹H NMR (CDCl₃) : δ 2.86 (3H, s), 3.39-3.43 (4H, m), 3.91-3.93 (2H, m), 3.99-4.01 (2H, m), 7.11-7.13 (1H, d), 7.44-7.59 (4H, m), 7.95-7.96 (2H, d), 8.14 (1H, s), 8.20-8.22 (1H, dd)
MS : C₂₂H₂₀F₃N₃O₃S requires 463; Found 464 (MH⁺)

### Example 1

### 4-(3-Phenylsulfonyl-1H-pyrrolo[3,2-b]pyridin-7-yl)-piperazine hydrochloride (E1)

3-Phenylsulfonyl-7-(4-*tert*-butyloxycarbonyl)piperazin-1-yl-1*H*-pyrrolo[3,2-b] pyridine (D4) (51 mg, 0.12 mmol) was taken up in 4M HCl (5 ml) and heated to 60 °C for 60min. The solvent was evaporated *in vacuo* to give the product as a white solid (E1) (39.8 mg)
NMR (CD₃OD) : δ_{H} 3.48-3.53 (4H, m), 4.05-4.08 (4H, m), 7.18-7.20 (1H, d), 7.58-7.69 (3H, m), 8.12-8.15 (2H, m), 8.28-8.30 (1H, d), 8.47 (1H, s).
Mass Spectrum: C₁₇H₁₈N₄O₂S requires 342; found: 343 (MH⁺)

### Example 2

### 4-Methyl-7-piperazin-1-yl-3-phenylsulfonyl-4H-pyrrolo[3,2-b] pyridine (E2)

7-(4-*tert*-butyloxycarbonyl)piperazin-1-yl-4-methyl-3-phenylsulfonyl-1*H*-pyrrolo [3,2-*b*] pyridine (D5) was taken up in 4M HCl (1 ml) and heated to 60 °C for 1 h. Solvents were evaporated *in vacuo* to yield a brown solid (E2) (6.9 mg)
NMR (CD₃OD): δ_{H} 3.53-3.55 (4H, m), 4.01-4.04 (4H, m), 4.28 (3H, s), 7.18-7.20 (1H, d), 7.60-7.75 (3H, m), 7.98-8.00 (2H, d), 8.27-8.29 (1H, d), 8.46 (1H, s)
Mass Spectrum: C₁₈H₂₀N₄O₂S requires 356; found 357 (MH⁺)

### Example 3

### 1-Methyl-7-piperazin-1-yl-3-phenylsulfonyl-1H-pyrrolo[3,2-b] pyridine (E3)

7-(4-*tert*-butyloxycarbonyl)piperazin-1-yl-1-methyl-3-phenylsulfonyl-1*H*-pyrrolo [3,2-*b*] pyridine (D6) (58.9 mg) was taken up in 4M HCl (6 ml) and heated to 60 °C for 60 min. Solvents evaporated *in vacuo* to yield an orange solid (E3) (50.2 mg)
NMR (CD₃OD) : δ_{H} 3.54 (4H, br s), 3.76 (4H, br s), 4.20 (3H, s), 7.44-7.46 (1H, d), 7.56-7.69 (3H, m), 8.11-8.14 (2H, d), 8.47-8.49 (1H, d), 8.60 (1H, s)
Mass Spectrum: C₁₈H₂₀N₄O₂S requires 356; found 357 (MH⁺)

The following Examples (E4-E5) were prepared using an analogous method to that used for Examples E1-E3.

### Example 4

### 1-Methyl-7-piperazin-1-yl-3-(2-fluorophenyl)sulfonyl-1H-pyrrolo[3,2-b] pyridine (E4)

Mass Spectrum: C₁₈H₁₉FN₄O₂S requires 374; found 375 (MH⁺)

### Example 5

### 4-Methyl-7-piperazin-1-yl-3-(2-fluorophenyl)sulfonyl-4H-pyrrolo[3,2-b] pyridine (E5)

Mass Spectrum: C₁₈H₁₉FN₄O₂S requires 374; found 375 (MH⁺)

### Example 6

### 7-Piperazin-1-yl-3-phenylsulfonyl-1H-pyrrolo[2,3-c] pyridine hydrochloride (E6)

A solution of 7-(4-*tert*-Butyloxycarbonyl)piperazin-1-yl-3-phenylsulfonyl-1*H*-pyrrolo[2,3-*c*] pyridine (D10) (55 mg, 0.124 mmol) in 4M HCl (3 ml) and 1,4-dioxane (3 ml) was heated at 60 °C for 1 h. After this period, reaction mixture was cooled and the solvents evaporated *in* vacuo to give the product as an off-white solid (E6) (54 mg)
NMR (DMSO-d₆) : δ_{H} 3.25-3.29 (4H, m), 3.64-3.68 (4H, m), 7.47 (1H, d, J = 5.9 Hz), 7.57-7.67 (3H, m), 7.89 (1H, d, J = 6 Hz), 8.00-8.02 (2H, m), 8.49 (1H, m) 9.17 (2H, br s) Mass Spectrum: C₁₇H₁₈N₄O₂S requires 342; Found 343 (MH⁺)

The following compounds of Examples E7-E8 were prepared in an analogous manner to Example 6:

### Example 7

### 3-(2-Fluorophenyl)sulfonyl-7-piperazin-1-yl-1H-pyrrolo[2,3-c]pyridine hydrochloride (E7)

Mass Spectrum: C₁₇H₁₇FN₄O₂S requires 360; Found 361 (MH⁺)

### Example 8

### 3-(3-Fluorophenyl)sulfonyl-7-piperazin-1-yl-1H-pyrrolo[2,3-c]pyridine hydrochloride (E8)

Mass Spectrum: C₁₇H₁₇FN₄O₂S requires 360; Found 361 (MH⁺)

### Example 9

### 2-Methyl-4-piperazin-1-yl-7-phenylsulfonylquinoline hydrochloride (E9)

To a stirred solution of 4-(4-*tert*-butyloxycarbonyl)piperazin-1-yl-2-methyl-7-phenylsulfonylquinoline (D14) (60 mg, 0.128 mmol) in dichloromethane (10 ml) was added trifluoroacetic acid (2 ml) dropwise. Reaction mixture was stirred for 1 h and solvents evaporated *in vacuo* and partitioned between dichloromethane and saturated aqueous K₂CO₃. Aqueous layer was re-extracted with dichloromethane (x 2) and the combined organic layers dried (Na₂SO₄) and the solvents evaporated *in vacuo* to give a colourless oil. This material was dissolved in dichloromethane / MeOH and treated with 1.1 eq. of 1*M* HCl in Et₂O. The solvents were evaporated *in vacuo* to give the title compound as a pale yellow solid (E9) (45 mg)
NMR (DMSO-d₆) : δ_{H} 2.67 (3H, s), 3.30-3.60 (8H, br m), 7.22 (1H, s), 7.64-7.75 (4H, m), 7.89-7.91 (1H, d, J = 8.6 Hz), 8.05 (3H, d, J = 7.5 Hz), 8.25 (1H, d, J = 8.7 Hz), 9.45 (2H, br s)
Mass Spectrum : C₂₀H₂₁N₃O₂S requires 353; Found 354 (MH⁺)

### Example 10

### 4-Phenylsulfonyl-8-piperazin-1-yl quinoline (E10)

8-(4-tert-Butyloxycarbonyl-piperazin-1-yl)-4-phenylsulphonyl quinoline (D16) (51.2 mg, 0.11 mmol) was stirred with 20 % trifluoroacetic acid in DCM (10ml) for 1 h. Solvents were then evaporated *in vacuo* and the residue partitioned between DCM (10ml) and sat. NaHCO₃ (10 ml). Aqueous layer re-extracted and the combined organic layers washed with brine (10 ml), dried (MgSO₄) and the solvents evaporated *in vacuo.* Purification by sep-pak chromatography (MeOH / NH₃/ DCM) gave the title product as a yellow solid (25.7 mg).
¹H NMR (CDCl₃) : δ 3.25-3.27 (4H, m), 3.38-3.40 (4H, m), 7.18-7.19 (1H, dd), 7.50-7.59 (4H, m), 7.97-7.99 (2H, dd), 8.18-8.19 (1H,d), 8.21-8.24 (1H, dd), 9.07-9.08 (1H, s)
MS : C₁₉H₂₇N₃SO₄ requires 353; found 354 (MH⁺)

### Example 11

### 4-Phenylsulfonyl-8-piperazin-1-yl-quinoline hydrochloride (E11)

4-Phenylsulfonyl-8-piperazin-1-yl-quinoline (E10) (25.7 mg, 0.75 mmol) was dissolved in DCM (5ml) and 1 M HCl in ether (80 µL, 0.80 mmol) was added. Solvent evaporated in *vacuo* to give the title product as an orange solid (26.9 mg).
¹H NMR : δ 3.73 (8H, bs), 7.55-7.75 (5H, m), 8.00-8.02 (2H, d), 8.38-8.39 (1H, d), 8.50-8.52 (1H, d), 9.35-9.35 (1H, d), 10.15 (2H, bs)

### Example 12

### 2-Methyl -4-phenylsulfonyl-8-piperazin-1-yl-quinoline (E12)

2-Methyl-4-phenylsulfonyl-8-(4-trifluoroacetyl-piperazin-1-yl) quinoline (D23) (106 mg, 0.23 mmol) was dissolved in MeOH (5 ml) and water (1.5 ml). K₂CO₃ was added and the solution stirred for 90 min. The solvent was evaporated *in vacuo* and the residue partitioned between DCM / MeOH (10 ml) and water (10 ml). The aqueous layer was re-extracted with DCM / MeOH (10 ml) and the combined organics washed with brine (20 ml), dried (MgSO₄) and the solvent evaporated *in vacuo* to give the title product as an orange oil (88 mg).
¹H NMR (CDCl₃) : δ 2.86 (3H, s), 3.17-3.20 (4H, m), 3.30-3.34 (4H, m), 7.11-7.14 (1H, dd), 7.40-7.57 (4H, m), 7.94-7.97 (2H, m), 8.10-8.13 (2H, m)
MS : C₂₀H₂₁N₃O₂S requires 367; Found 368 (MH⁺)

### Example 13

### 2-Methyl-4-phenylsulfonyl-8-piperazin-1-ylquinoline hydrochloride (E13)

2-Methyl -4-phenylsulfonyl-8-piperazin-1-yl-quinoline (E12) (88 mg, 0.23 mmol) was taken up in DCM and 1*M* HCl in ether (0.274 ml) added. Solvents evaporated *in vacuo* to give the title product as a brown solid (93 mg).
¹H NMR (CDCl₃): δ 3.06 (3H, s), 3.92 (8H, bs), 7.56-7.59 (2H, m), 7.64-7.73 (2H, m), 8.00-8.01 (2H, d), 8.24 (1H, bs), 8.28 (1H, s), 8.58-8.60 (1H, d), 10.35 (2H, bs)
MS : C₂₀H₂₁N₃O₂S requires 367; Found 368 (MH⁺)

### Examples 14-16

Examples 14-16 were prepared in an analogous manner to Example 12

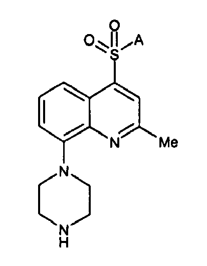

| **Example** | **A** | **MH**^{**+**} | **Formula** |
|---|---|---|---|
| **14** | 2-fluorophenyl | 386 | C₂₀H₂₀FN₃O₂S |
| **15** | 3-fluorophenyl | 386 | C₂₀H₂₀FN₃O₂S |
| **16** | 3-chlorophenyl | 402 | C₂₀H₂₀ClN₃O₂S |

### Pharmacological data

Compounds can be tested following the procedures outlined in WO98/27081. The compounds of Examples E1-E16 were tested and showed good affinity for the 5-HT₆ receptor, having pKi values > 7.0 at human cloned 5-HT₆ receptors. In particular, Examples E6-E16 exhibited pKi values > 7.5 and Examples E9-E16 exhibited pKi values > 8.0.

## Claims

1. A compound of formula (I) or a pharmaceutically acceptable salt thereof: 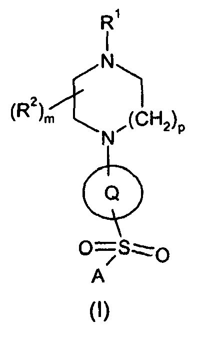 wherein
R¹ and R² independently represent hydrogen or C₁₋₆ alkyl or R¹ is linked to R² to form a group (CH₂)₂, (CH₂)₃ or (CH₂)₄;
p represents 1 or 2;
m represents an integer from 1 to 4, when m is an integer greater than 1, two R² groups may instead be linked to form a group CH₂, (CH₂)₂ or (CH₂)₃;
Q represents a group of formula (i), (ii), (iii) or (iv): 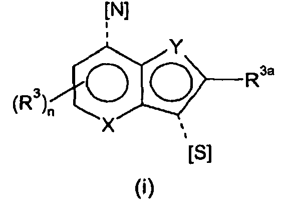 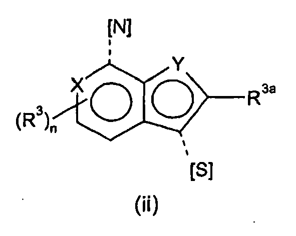 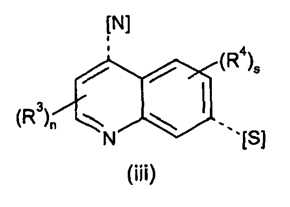 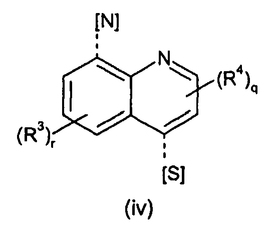 wherein [N] and [S] represent the attachment points for the groups 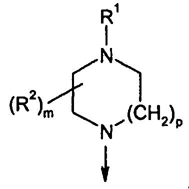 and 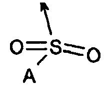 respectively;
one of X and Y represents -N= and the other represents -N(R⁵)-;
R³ and R⁴ independently represent hydrogen, halogen, cyano, -CF₃, -OCF₃, C₁₋₆ alkyl, C₁₋₆ alkoxy, C₁₋₆ alkanoyl or a group -CONR⁶R⁷;
R^{3a} and R⁵ independently represent hydrogen or C₁₋₆ alkyl;
R⁶ and R⁷ independently represent hydrogen or C₁₋₆ alkyl or together may be fused to form a 5- to 7- membered aromatic or non-aromatic heterocyclic ring optionally interrupted by an O or S atom;
n and q independently represent 1 or 2;
r and s independently represent an integer from 1 to 3;
A represents a group -Ar¹ or - Ar²Ar³;
Ar¹, Ar² and Ar³ independently represent an aryl group or a heteroaryl group, both of which may be optionally substituted by one or more (eg. 1, 2 or 3) substituents which may be the same or different, and which are selected from the group consisting of halogen, hydroxy, cyano, nitro, trifluoromethyl, trifluoromethoxy, C₁₋₆ alkyl, trifluoromethanesulfonyloxy, pentafluoroethyl, C₁₋₆ alkoxy, arylC₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, arylsulfonyl, arylsulfonyloxy, arylsulfonyl C₁₋₆ alkyl, C₁₋₆ alkylsulfonamido, C₁₋₆ alkylamido, C₁₋₆ alkylsulfonamido C₁₋₆ alkyl, C₁₋₆ alkylamido C₁₋₆ alkyl, arylsulfonamido, arylcarboxamido, arylsulfonamidoC₁₋₆ alkyl, arylcarboxamido C₁₋₆ alkyl, aroyl, aroyl C₁₋₆ alkyl, arylC₁₋₆ alkanoyl, or a group CONR⁸R⁹ or SO₂NR⁸R⁹, wherein R⁸ and R⁹ independently represent hydrogen or C₁₋₆ alkyl or together may be fused to form a 5- to 7- membered aromatic or non-aromatic heterocyclic ring optionally interrupted by an O or S atom;
or solvates thereof.

2. A compound according to claim 1 which is a compound of formula E1-E16 or a pharmaceutically acceptable salt thereof.

3. A pharmaceutical composition which comprises a compound according to claim 1 or claim 2 and a pharmaceutically acceptable carrier or excipient.

4. A compound according to claim 1 or claim 2 for use in therapy.

5. A compound according to claim 1 or claim 2 for use in the treatment of depression, anxiety, obesity and cognitive memory disorders.

6. Use of a compound according to claim 1 or claim 2 in the manufacture of a medicament for the treatment of depression, anxiety, obesity and cognitive memory disorders.

## Patentansprüche

1. Verbindung der Formel (I) oder deren pharmazeutisch annehmbares Salz: 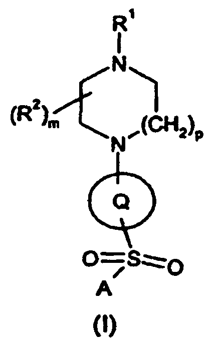 worin gilt:
R¹ und R² sind unabhängig Wasserstoff oder C₁₋₆-Alkyl oder R¹ ist mit R² verbunden, um eine Gruppe (CH₂)₂, (CH₂)₃ oder (CH₂)₄ zu bilden;
p ist 1 oder 2;
m ist eine ganze Zahl von 1 bis 4, und wenn m eine ganze Zahl ist, die größer als 1 ist, können zwei R²-Gruppen stattdessen verbunden sein, um eine Gruppe CH₂, (CH₂)₂ oder (CH₂)₃ zu bilden;
Q ist eine Gruppe der Formel (i), (ii), (iii) oder (iv): 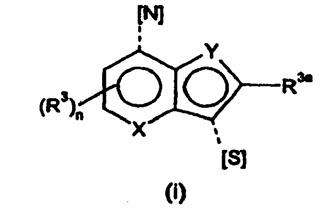 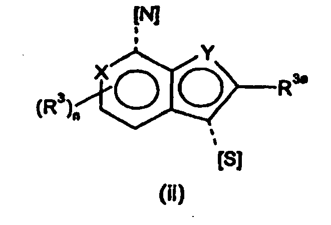 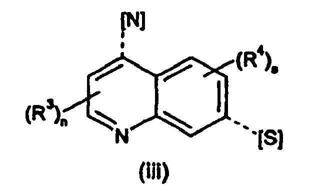 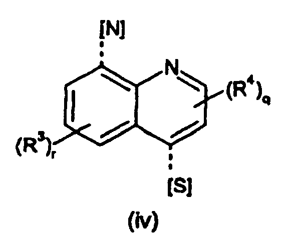 worin [N] und [S] die Anheftungspunkte für die Gruppen 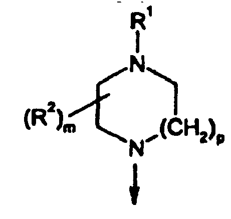 bzw. 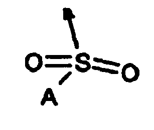 sind;
eines aus X und Y ist -N= und das andere ist -N(R⁵)-;
R³ und R⁴ sind unabhängig Wasserstoff, Halogen, Cyano, -CF₃, -OCF₃, C₁₋₆-Alkyl, C₁₋₆-Alkoxy, C₁₋₆-Alkanoyl oder eine Gruppe -CONR⁶R⁷;
R^{3a} und R⁵ sind unabhängig Wasserstoff oder C₁₋₆-Alkyl;
R⁶ und R⁷ sind unabhängig Wasserstoff oder C₁₋₆-Alkyl oder können zusammen verbunden sein, um einen 5- bis 7-gliedrigen aromatischen oder nicht-aromatischen heterocyclischen Ring zu bilden, der gegebenenfalls durch ein O- oder S-Atom unterbrochen ist;
n und q sind unabhängig 1 oder 2;
r und s sind unabhängig eine ganze Zahl von 1 bis 3;
A ist eine Gruppe -Ar¹ oder -Ar²Ar³;
Ar¹, Ar² und Ar³ sind unabhängig eine Aryl-Gruppe oder eine Heteroaryl-Gruppe, von denen beide gegebenenfalls mit einem oder mehreren (z.B. 1, 2 oder 3) Substituenten substituiert sein können, die gleich oder unterschiedlich sein können und die aus der Gruppe ausgewählt sind, die besteht aus Halogen, Hydroxy, Cyano, Nitro, Trifluormethyl, Trifluormethoxy, C₁₋₆-Alkyl, Trifluormethansulfonyloxy, Pentafluorethyl, C₁₋₆₋Alkoxy, Aryl-C₁₋₆-alkoxy, C₁₋₆-Alkylthio, C₁₋₆-Alkoxy-C₁₋₆-alkyl, C₃₋₇₋Cycloalkyl-C₁₋₆-alkoxy, C₁₋₆-Alkanoyl, C₁₋₆-Alkoxycarbonyl, C₁₋₆-Alkylsulfonyl, C₁₋₆-Alkylsulfinyl, C₁₋₆-Alkylsulfonyloxy, C₁₋₆-Alkylsulfonyl-C₁₋₆-alkyl, Arylsulfonyl, Arylsulfonyloxy, Arylsulfonyl-C₁₋₆-alkyl, C₁₋₆₋Alkylsulfonamido, C₁₋₆-Alkylamido, C₁₋₆-Alkylsulfonamido-C₁₋₆-alkyl, C₁₋₆₋Alkylamido-C₁₋₆-alkyl, Arylsulfonamido, Arylcarboxamido, Arylsulfonamido-C₁₋₆-alkyl, Arylcarboxamido-C₁₋₆-alkyl, Aroyl, Aroyl-C₁₋₆-alkyl, Aryl-C₁₋₆₋alkanoyl oder eine Gruppe CONR⁸R⁹ oder SO₂NR⁸R⁹, worin R⁸ und R⁹ unabhängig Wasserstoff oder C₁₋₆-Alkyl sind oder zusammen verschmolzen sein können, um einen 5- bis 7-gliedrigen aromatischen oder nicht-aromatischen heterocyclischen Ring, der gegebenenfalls durch ein O- oder S-Atom unterbrochen ist, zu bilden;
oder Solvate davon.

2. Verbindung nach Anspruch 1, die eine Verbindung der Formel E1-E16 oder ein pharmazeutisch annehmbares Salz davon ist.

3. Pharmazeutische Zusammensetzung, die eine Verbindung nach Anspruch 1 oder Anspruch 2 und einen pharmazeutisch annehmbaren Träger oder Hilfsstoff umfaßt.

4. Verbindung nach Anspruch 1 oder Anspruch 2 zur Verwendung in der Therapie.

5. Verbindung nach Anspruch 1 oder Anspruch 2 zur Verwendung in der Behandlung von Depression, Angstzustand, Fettleibigkeit und kognitiven Erinnerungsstörungen.

6. Verwendung einer Verbindung nach Anspruch 1 oder nach Anspruch 2 zur Herstellung eines Medikamentes zur Behandlung von Depression, Angstzustand, Fettleibigkeit und kognitiven Erinnerungsstörungen.

## Revendications

1. Composé de formule (I) ou sel de celui-ci acceptable du point de vue pharmaceutique : 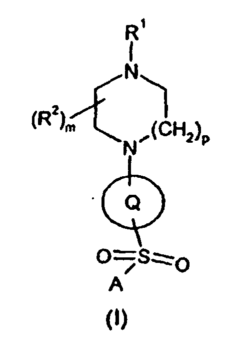 dans laquelle :
R¹ et R² représentent indépendamment un atome d'hydrogène ou un groupe alkyle en C₁₋₆ ou bien R¹ est relié à R² pour former un groupe (CH₂)₂, (CH₂)₃ ou (CH₂)₄ ;
p représente 1 ou 2 ;
m représente un entier de 1 à 4, lorsque m est un entier supérieur à 1, deux groupes R² peuvent au lieu de cela être liés pour former un groupe CH₂, (CH₂)₂ ou (CH₂)₃ ;
Q représente un groupe de formule (i), (ii), (iii) ou (iv) : 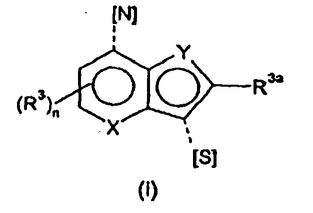 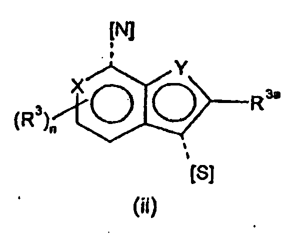 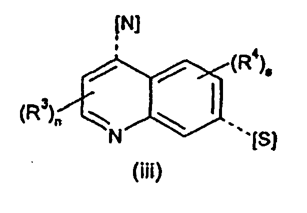 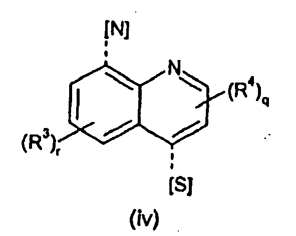 dans lesquelles [N] et [S] représentent les points d'attachement pour les groupes 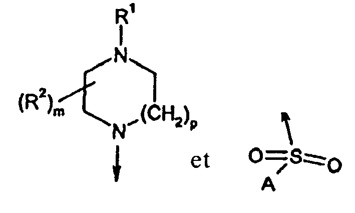 respectivement, l'un des X et Y représente -N= et l'autre représente -N(R⁵)- ;
R³ et R⁴ représentent indépendamment un atome d'hydrogène, d'halogène, un groupe cyano, -CF₃, -OCF₃, alkyle en C₁₋₆, alcoxy en C₁₋₆, alcanoyle en C₁₋₆ ou un groupe -CONR⁶R⁷ ;
R^{3a} et R⁵ représentent indépendamment un atome d'hydrogène, ou un groupe alkyle en C₁₋₆ ;
R⁶ et R⁷ représentent indépendamment un atome d'hydrogène ou un groupe alkyle en C₁₋₆ ou bien peuvent ensemble être condensés pour former un noyau hétérocyclique aromatique ou non aromatique de 5 à 7 chaînons interrompu éventuellement par un atome d'oxygène ou de soufre ;
n et q représentent indépendamment 1 ou 2 ;
r et s représentent indépendamment un entier de 1 à 3 ;
A représente un groupe -Ar¹ ou -Ar²Ar³ ;
Ar¹, Ar² et Ar³ représentent indépendamment un groupe aryle ou un groupe hétéroaryle, qui peuvent tous les deux être éventuellement substitués par un ou plusieurs (par exemple, 1, 2 ou 3) substituants qui peuvent être identiques ou différents, et qui sont choisis dans le groupe consistant en atome d'halogène, groupe hydroxy, cyano, nitro, trifluorométhyle, trifluorométhoxy, alkyle en C₁₋₆, trifluorométhanesulfonyloxy, pentafluoroéthyle, alcoxy en C₁₋₆, arylalcoxy en C₁₋₆, alkylthio en C₁₋₆, alcoxy(en C₁₋₆)alkyle en C₁₋₆, cycloalkyl(en C₃₋₇)alcoxy en C₁₋₆, alcanoyle en C₁₋₆, alcoxycarbonyle en C₁₋₆, alkylsulfonyle en C₁₋₆, alkylsulfinyle en C₁₋₆, alkylsulfonyloxy en C₁₋₆, alkyl(en C₁₋₆)sulfonylalkyle en C₁₋₆, arylsulfonyle, arylsulfonyloxy, arylsulfonylalkyle en C₁₋₆, alkylsulfonamido en C₁₋₆, alkylamido en C₁₋₆, alkyl(en C₁₋₆)sulfonamidoalkyle en C₁₋₆, alkyl(en C₁₋₆)amidoalkyle en C₁₋₆, arylsulfonamido, arylcarboxamido, arylsulfonamidoalkyle en C₁₋₆, arylcarboxamidoalkyle en C₁₋₆, aroyle, aroylalkyle en C₁₋₆, arylalcanoyle en C₁₋₆, ou un groupe CONR⁸R⁹ ou SO₂NR⁸R⁹, où R⁸ et R⁹ représentent indépendamment un atome d'hydrogène ou un groupe alkyle en C₁₋₆, ou bien peuvent ensemble être condensés pour former un noyau hétérocyclique aromatique ou non aromatique de 5 à 7 chaînons éventuellement interrompu par un atome d'oxygène ou de soufre ;
ou solvates de celui-ci.

2. Composé selon la revendication 1, qui est un composé de formule E1-E16 ou un sel de ceux-ci acceptable du point de vue pharmaceutique.

3. Composition pharmaceutique qui comprend un composé selon la revendication 1 ou la revendication 2 et un support ou excipient acceptable du point de vue pharmaceutique.

4. Composé selon la revendication 1 ou la revendication 2 utile en thérapie.

5. Composé selon la revendication 1 ou la revendication 2 utile dans le traitement de la dépression, de l'anxiété, de l'obésité et des troubles cognitifs de la mémoire.

6. Utilisation d'un composé selon la revendication 1 ou la revendication 2 dans la fabrication d'un médicament destiné au traitement de la dépression, de l'anxiété, de l'obésité et des troubles cognitifs de la mémoire.
